# FASCICULE DE BREVET EUROPEEN

(11) **EP 0 426 545 B1**
(45) Date de publication et mention de la délivrance du brevet: **25.08.1993**
(21) Numéro de dépôt: 90403032.7
(22) Date de dépôt: 26.10.1990
(51) Int. Cl.: A61F 2/00, A61F 2/48

(54) **Dispositif de commande pour un sphincter artificiel, et prothèse implantable comprenant ledit dispositif**
Steuereinrichtung für einen künstlichen Schliessmuskel und implantierbare Prothese mit dieser Vorrichtung
Control device for an artificial sphincter and implantable prothesis comprising this device

(30) Priorité: 03.11.1989 FR 8914402
(43) Date de publication de la demande: 08.05.1991
(73) Titulaire: SYNTHELABO, 92350 Le Plessis Robinson (FR)
(72) Inventeur: Bailly, Pierre, F-92120 Montrouge (FR)
(74) Mandataire: Bonnetat, Christian

(56) Documents cités:
- DE-A- 2 614 227
- US-A- 4 419 985
- US-A- 4 571 749

## Description

La présente invention concerne un dispositif de commande pour un sphincter artificiel d'un conduit naturel, notamment urinaire, ainsi qu'une prothèse implantable comprenant ledit dispositif de commande.

L'un des moyens connus pour remédier aux inconvénients de l'incontinence urinaire consiste à placer autour de l'urètre (chez l'homme) ou autour du col vésical (chez la femme) un sphincter artificiel, qui est un collier, ou manchette, et qui, à la demande, peut comprimer le conduit urinaire, se substituant en cela à l'appareil sphinctérien naturel défaillant.

De telles manchettes sont bien connues, et fonctionnent de façon satisfaisante. On en trouve des descriptions par exemple dans les brevets US-3 863 622, US-4 222 377 et US-4 632 114. Elles consistent par exemple en un ballonnet de forme générale torique, entouré d'une ceinture souple ou rigide, l'ensemble lui-même venant entourer le conduit urinaire. Selon la pression de fluide hydraulique qui règne à l'intérieur du ballonnet, le conduit naturel se trouve comprimé (état de continence) ou non (phase de miction).

On connaît également des dispositifs de commande de sphincters artificiels, qui permettent d'injecter dans la manchette un fluide en vue de comprimer le conduit urinaire ou, au contraire, d'en évacuer du fluide, pour relâcher le conduit et permettre la miction. Ces dispositifs sont également implantables, par exemple dans l'abdomen du patient, et peuvent être manupulés à travers la peau.

Or, d'une part, et en l'absence de tout dispositif matériel traversant la peau, la seule manipulation simple qu'on puisse effectuer à travers la peau est une pression, exercée par exemple avec un doigt, sur un dispositif de commande implanté. D'autre part, l'état de continence est le plus fréquent, de sorte que le sphincter artificiel doit rester sous pression de façon prolongée, n'étant relâché que lors des phases de miction.

Ainsi, on connaît déjà, par le brevet US-4 571 749, qui décrit un dispositif conforme au préambule de la revendication 1, un système de sphincter urinaire implantable dont le dispositif de commande comprend un soufflet, relié à une enceinte, remplie de fluide incompressible, comportant une membrane souple. Lorsque le patient presse vigoureusement avec ses doigts sur ladite membrane, cela provoque l'allongement du soufflet ce qui, à son tour, entraîne l'augmentation de volume d'une chambre, remplie de fluide incompressible, reliée au sphincter, en réduisant ainsi la pression dans ledit sphincter. Au contraire, quand aucune pression n'est exercée sur la membrane, la force de rappel exercée par un diaphragme constituant une des parois de ladite chambre tend à ramener cette dernière à son volume initial, en assurant ainsi la fonction de compression du sphincter.

Cependant, ce dispositif présente un certain nombre d'inconvénients. Tout d'abord, l'action du patient sur la membrane peut ne pas se traduire par un allongement suffisant du soufflet en raison de la conformation même de la membrane et du fait que ladite enceinte elle-même peut se dilater plutôt que le soufflet. Par ailleurs, la surface d'extrémité du soufflet, agissant sur la paroi correspondante de la chambre reliée au sphincter, est restreinte, ce qui ne garantit pas une action efficace dudit soufflet sur ladite chambre. De plus, la structure du dispositif de commande, décrit dans le brevet US-4 571 749, est complexe, et donc coûteuse et peu fiable.

La présente invention a pour but d'éviter ces inconvénients, et concerne un dispositif de commande pour sphincter artificiel dont la structure est simple, fiable et économique.

A cet effet, le dispositif de commande pour un sphincter artificiel d'un conduit naturel, notamment urinaire, ledit sphincter comportant une enveloppe de volume variable, du type comprenant une première chambre, contenant un fluide incompressible, en communication avec un organe d'actionnement permettant de faire varier le volume de ladite première chambre, une deuxième chambre, contenant un fluide incompressible, en communication avec ledit sphincter, lesdites première et deuxième chambres étant agencées de sorte qu'un variation de volume de ladite première chambre entraîne une variation de volume, de même sens, de ladite deuxième chambre, ledit dispositif pouvant passer, par actionnement dudit organe, d'un premier état, dans lequel les volumes desdites première et deuxième chambres sont minimaux, le sphincter obturant alors ledit conduit naturel, à un second état, dans lequel les volumes desdites première et deuxième chambres sont maximaux, le sphincter ouvrant alors ledit conduit, des moyens élastiques de rappel étant prévus pour ramener spontanément ledit dispositif dudit second état audit premier état, est remarquable, selon l'invention en ce que ladite première chambre est un ballonnet, en ce que ladite deuxième chambre est définie par deux plateaux, reliés par une membrane formant soufflet, entre lesquels est logé ledit ballonnet, et en ce que lesdits moyens élastiques de rappel comprennent au moins une bague élastique entourant lesdits plateaux.

Ainsi, le fait de réaliser la première chambre sous forme d'un ballonnet, qui réagit à toute sollicitation de l'organe d'actionnement par le patient, et, en outre, le fait de loger ledit ballonnet entre les deux plateaux de la deuxième chambre, avec lesquels il est en contact étroit sur une grande partie de sa surface, garantissent une parfaite efficacité du dispositif de commande de l'invention. En particulier, on conçoit qu'une augmentation déterminée du volume du ballonnet se traduit directement par une augmentation correspondante du volume de la deuxième chambre.

Avantageusement, lesdites première et deuxième chambres sont logées dans un boîtier rigide ou semi-rigide communiquant avec un ballon de réserve par un orifice ménagé dans la paroi du boîtier en définissant ainsi une troisième chambre, et il est prévu entre lesdites deuxième et troisième chambres des moyens de liaison deuxième et troisième chambres lorsque lesdits plateaux sont rapprochés l'un de l'autre, et d'isoler lesdites deuxième et troisième chambres l'une de l'autre lorsque lesdits plateaux sont éloignés l'un de l'autre.

Plus particulièrement, lesdits moyens de liaison fluidique entre lesdites deuxième et troisième chambres comprennent un orifice, ménagé dans l'un desdits plateaux, pouvant être ouvert ou fermé grâce à un clapet mobile qui ferme le passage lorsque lesdits plateaux sont écartés l'un de l'autre, et qui ouvre le passage, en venant buter contre une tige solidaire de l'autre desdits plateaux, lorsque lesdits plateaux se rapprochent l'un de l'autre.

De préférence, lesdits plateaux présentent, sur leurs faces en regard l'une de l'autre, des reliefs assurant le positionnement du ballonnet.

En particulier, le ballonnet ayant une forme sensiblement cylindrique à l'état gonflé, l'un des plateaux comporte un évidement en forme de gouttière tandis que l'autre desdits plateaux présente un relief de forme convexe conjuguée à celle dudit évidement, pour le positionnement dudit ballonnet.

Avantageusement, ledit organe d'actionnement comporte un réservoir souple de fluide incompressible.

Par ailleurs, le dispositif de commande peut comprendre un organe de blocage permettant d'interrompre, temporairement, la communication fluidique entre ledit sphincter et ladite deuxième chambre. Ledit organe de blocage comprend, avantageusement, un corps en matériau élastique contenant deux chambres sensiblement sphériques en communication l'un avec l'autre, et une bille susceptible d'occuper l'une ou l'autre desdites chambres, et ledit corps peut être solidaire dudit organe d'actionnement.

Les figures du dessin annexé feront bien comprendre comment l'invention peut être réalisée.

Les figures 1 et 2 illustrent le principe du fonctionnement d'une prothèse comprenant un sphincter artificiel et un dispositif de commande.

Les figures 3 à 12 illustrent en détail un mode de réalisation d'une telle prothèse, et en particulier du dispositif de commande.

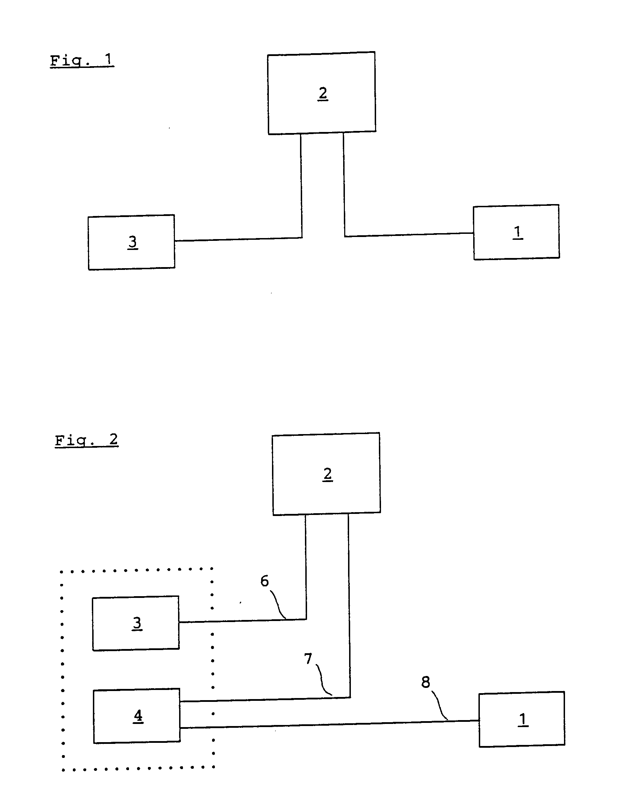

Le schéma de la figure 1 montre une prothèse comprenant un sphincter artificiel, ou manchette 1, relié par un tube à un dispositif de commande 2, lui-même relié par un tube à un organe d'actionnement 3.

Le schéma de la figure 2 montre une prothèse comportant, entre le dispositif de commande 2 et la manchette 1, un dispositif 4 de blocage de la circulation du fluide. Ce dispositif 4 fonctionne en "tout ou rien", à la manière d'un interrupteur ou d'un coupe-circuit. Il permet de maintenir la manchette 1 dans un état permanent, quelle que soit l'action exercée sur l'organe d'actionnement 3. En particulier, il permet de laisser la manchette en état de dépression permanente, après une pression sur l'organe d'actionnement, pour que la patient reste constamment incontinent, par exemple pendant la période de cicatrisation après mise en place de la prothèse, ou pendant un examen endoscopique.

Comme l'organe d'actionnement 3, le bloqueur 4 est implantable, et manipulable à travers la peau. Pour une question de commodité, en particulier lors de l'implantation de la prothèse, il peut être rendu solidaire de l'organe d'actionnement 3, comme cela est symbolisé par le rectangle en pointillés de la figure 2.

L'ensemble de la prothèse comprend donc trois composants qui sont la manchette 1, le dispositif de commande 2, et l'organe d'actionnement et de bloquage 3, 4 les trois composants étant reliés entre eux par des tubes souples 6, 7, 8, comme ilustré par le schéma de la figure 2.

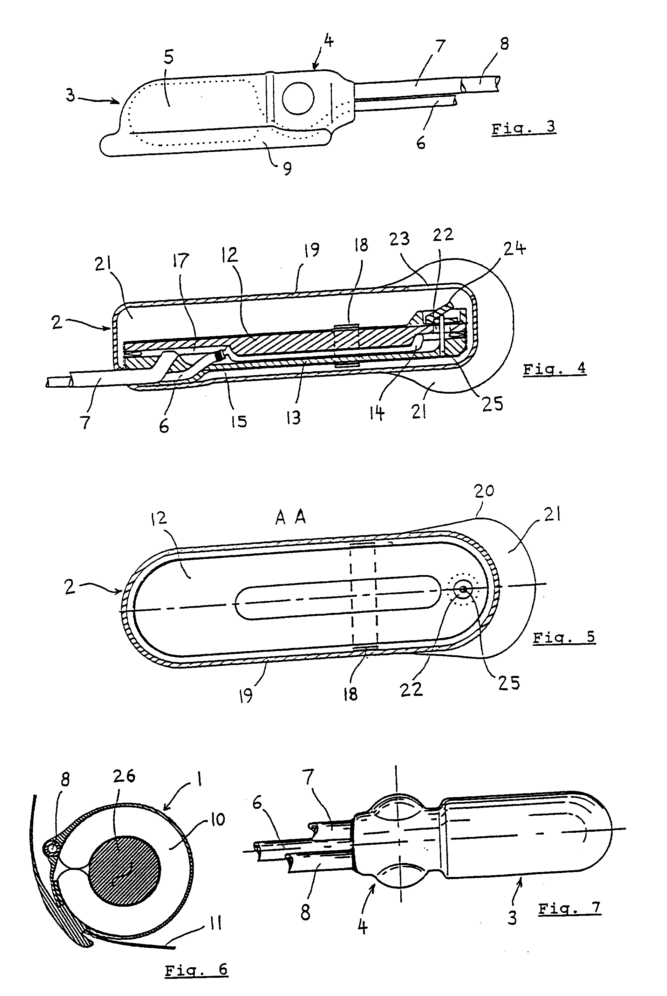

Les figures 3, 7 et 8 illustrent un mode de réalisation de l'organe d'actionnement et de bloquage 3, 4.

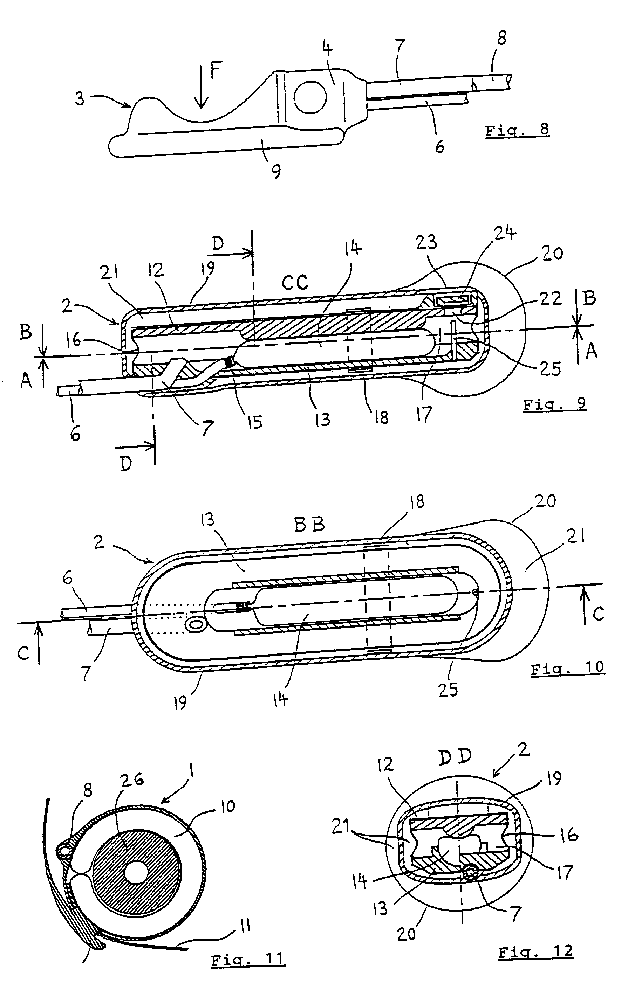

L'organe d'actionnement 3 est constitué essentiellement d'une chambre ou réservoir 5 en matière souple, relié au dispositif de commande 2 par un tube souple 6. Une force F appliquée sur la chambre 5 écrase celle-ci (figure 8) de sorte qu'un partie du fluide qu'elle contient est refoulée par le tube 6. L'annulation de la force F permet le retour à l'état initial.

Le dispositif de blocage 4 est constitué d'un corps en matériau élastique contenant deux chambres sphériques empiétant l'une sur l'autre, et dont l'une est occupée par une bille. Selon que la bille se trouve dans l'une ou l'autre des chambres sphériques, un fluide hydraulique peut, ou ne peut pas, circuler entre les tubes 7, 8 d'entrée/sortie du bloqueur 4. ladite bille peut passer une chambre à l'autre par l'effet d'une manipulation du bloqueur à travers la peau.

Un bloqueur hydraulique tel que celui mis en oeuvre dans la présente invention est décrit en détail dans la demande de brevet français n° 89 06 244.

Comme indiqué plus haut, le bloqueur 4 et l'organe d'actionnement 3 constituent, de préférence, un seul bloc fonctionnel. Comme on le voit sur les figures 3 et 8, ce dernier peut être avantageusement muni d'une semelle (ou socle) 9 ayant une surface sensiblement plane, laquelle pourra être placée au droit d'un support suffisamment ferme, pour que la force F exercée sur l'organe d'actionnement 3 déforme effectivement la chambre 5, sans que l'ensemble 3, 4 ne se déplace.

La figure 7 montre à titre de variante, un bloc fonctionnel 3, 4 dépourvu de socle 9.

Une manchette 1 utilisable selon l'invention est illustrée par les vues en coupe des figures 6 et 11. Une telle manchette comporte un tube souple 8 (vu en coupe transversale sur les figures 6 et 11), communiquant, d'une part, avec l'intérieur d'un ballonnet torique 10 et, d'autre part, soit directement avec un dispositif de commande comme illustré par la figure 1, soit, de préférence, avec le bloqueur 4, comme illustré par la figure 2. Le ballonnet 10 est, de préférence, préformé pour éviter la formation de plis lorsqu'on le gonfle, et il est lui-même entouré d'une ceinture inextensible 11 fermée par un système à boutons ou à crémaillère de type connu.

La figure 6 représente la manchette dans l'état où elle assure la continence, c'est-à-dire avec le ballonnet 10 gonflé et le conduit urinaire 26 comprimé, tandis que la figure 11 représente la manchette dans l'état où elle assure l'incontinence et permet la miction, c'est-à-dire avec le ballonnet 10 dégonflé et le conduit urinaire 26 non comprimé.

Le dispositif de commande 2 est illustré par les figures 4, 5, 9, 10 et 12, qui sont toutes des vues en coupe. Les figures 4 et 5 le représentent à l'état de continence, tandis que les figures 9, 10 et 12 le représentent lors de la phase de miction.

Le dispositif de commande selon l'invention comprend un plateau supérieur rigide 12 et un plateau inférieur rigide 13, entre lesquels est disposé un ballonnet 14 rempli de fluide incompressible et communiquant avec l'organe d'actionnement 3 par l'intermédiare du tube souple 6, grâce à une connexion 15 telle qu'une ligature, ledit tube 6 traversant le plateau inférieur 13.

Les deux plateaux 12, 13 vus par leurs faces principales (figures 5 et 10) ont la même forme, par exemple ovale, ou rectangle à coins arrondis. Ils sont disposés en regard l'un de l'autre et sont entourés d'une membrane souple formant soufflet 16. Cette membrane peut être simplement collée sur les chants des plateaux 12 et 13 mais, pour garantir une meilleure étanchéité, elle enveloppe de préférence complètement les deux plateaux, et n'est munie d'orifices qu'en regard des orifices correspondants ménagés dans les plateaux.

Les deux plateaux 12, 13 et le soufflet 16 délimitent une chambre 17, laquelle est partiellement occupée par le ballonnet 14. La chambre 17 est remplie de fluide hydraulique et communique soit (figure 1) directement avec la manchette, soit (figure 2) avec le bloqueur 4 au moyen d'un tube souple 7, traversant lui aussi le plateau inférieur 13.

L'ensemble des deux plateaux 12, 13 et du soufflet 16 est ceinturé par une bague élastique 18 qui tend à rapprocher lesdits plateaux, de manière à diminuer le volume de la chambre 17 et à écraser le ballonnet 14. Au contraire, lorsque du fluide hydraulique est injecté dans le ballonnet 14, celui-ci se gonfle et tend à écarter les deux plateaux 12, 13 de manière à augmenter le volume de la chambre 17.

L'ensemble des deux plateaux 12, 13 et du soufflet 16, contenant le ballonnet 14 et ceinturé par la bague 18, est lui-même contenu dans un boîtier 19, rigide ou semi-rigide, étanche, traversé par les tubes 6 et 7, et dont l'intérieur communique en permanence avec un ballon de réserve 20 grâce à un orifice 23 ménagé dans la paroi du boîtier 19. Le ballon 20 entoure partiellement le boîtier 19, et ils délimitent ensemble une chambre 21, également remplie de fluide hydraulique, et pouvant être mise en communication avec la chambre 17.

A cet effet, la chambre 17 est munie d'un moyen d'ouverture et de fermeture, débouchant dans la chambre 21, et dont l'état dépend de la position relative des deux plateaux 12 et 13. Ce moyen d'ouverture et de fermeture a pour fonction de mettre en communication les chambres 17 et 21 lorsque les deux plateaux 12 et 13 sont proches l'un de l'autre, et de les isoler l'une de l'autre lorsque les deux plateaux sont éloignés l'une de l'autre par l'effet du gonflement du ballonnet 14.

Ainsi, selon un mode de réalisation particulier, le plateau supérieur 12 est percé d'un orifice 22 qui peut être ouvert ou obturé grâce à un clapet mobile 24. Lorsque les deux plateaux 12 et 13 sont écartés, le clapet 24 ferme le passage 22. Lorsque le ballonnet 14 se vide et que les deux plateaux se rapprochent sous l'effet de la bague de rappel 18, le clapet 24 ouvre le passage en venant buter sur une tige 25, solidaire du plateau inférieur 13, qui l'écarte de sa position fermée, de sorte que du fluide hyraulique peut être échangé entre les chambres 17 et 21, et que les pressions y sont équilibrées.

Inversement, lorsque le ballonnet 14 se gonfle et tend à écarter les plateaux 12, 13, le clapet 24 s'éloigne de la tige 25 et reprend sa position de fermeture, et l'étanchéité entre les chambres 17 et 21 est assurée grâce à la dépression qui se crée dans la chambre 17, le clapet 24 se trouvant alors plus fortement plaqué sur l'orifice 22 de communication.

Les deux plateaux 12, 13 peuvent, en principe, être constitués de plaques ayant des faces planes. Toutefois, comme on le voit sur les figures 4, 5, 9, 10 et 12, les deux faces en regard l'une de l'autre des plateaux présentent avantageusement des reliefs destinés à mieux assurer la position correcte du ballonnet 14. Ainsi, l'un des plateaux, par exemple le plateau inférieur 13, peut comporter une cavité ou évidement, adapté à la forme du ballonnet 14, par exemple en forme de gouttière, lorsque ce dernier est de forme sensiblement cylindrique à l'état gonflé. Pour garantir un bon écrasement du ballonnet 14 par le plateau supérieur 12, ce dernier peut alors être muni d'un relief, ou bombement, de forme convexe conjuguée à celle de la cavité du plateau inférieur 13.

Le fonctionnement et la manipulation de la prothèse selon l'invention découlent de la description qui précède. Ils sont résumés et complétés par les explications qui vont suivre. On supposera que l'ensemble de la prothèse a été mis en place sur un patient, c'est-à-dire que la manchette 1 entoure l'urètre ou le col vésical, que le bloc fonctionnel 3, 4 est accessible au patient, et que le dispositif de commande 2 a été implanté dans l'abdomen, les trois composants étant reliés entre eux par des tubes souples selon le schéma de la figure 2.

En position de repos, c'est-à-dire à l'état de continence, (figures 3 à 6), aucune force F n'est exercée sur l'organe d'actionnement 3, le ballonnet 14 est dégonflé, les plateaux 12 et 13 sont pressés l'un contre l'autre par la bague 18, la chambre 17 a un volume minimal et, par l'intermédiaire du tube 7, du bloqueur 4 (en position communicante), et du tube 8, le ballonnet 10 de la manchette 1 est à son volume maximal et comprime donc le conduit urinaire 26. Le clapet mobile 24 est alors en position ouverte, les chambres 17 et 21 communiquent entre elles, et la pression qui y règne est aussi celle du ballonnet 10 de la manchette 1.

Grâce à la souplesse du ballon de réserve 20, toute variation de pression qui a lieu dans l'abdomen du patient est immédiatement transmise à la chambre 21, donc à la chambre 17 et au ballonnet 10 de la manchette 1.

Ainsi, en particulier, lorsque le patient contracte sa paroi abdominale, par exemple à l'occasion d'efforts, de toux, de rire, de hoquet, et., l'augmentation de pression qui agit sur la vessie (et qui tend donc à provoquer la fuite d'urine) provoque également une augmentation de la compression du conduit urinaire, de sorte que la continence reste assurée.

En phase de miction (figures 8 à 12), une force F est appliquée à la l'organe d'actionnement 3, du fluide hydraulique en est chassé par le tube 6 et vient remplir le ballonnet 14 du dispositif de commande 2. Ledit ballonnet 14 agit à la manière d'un vérin qui écarte les plateaux 12 et 14, l'orifice 22 se ferme grâce au clapet 24 et, du fait de l'augmentation du volume de la chambre 17, qui n'est plus en communication avec la chambre 21, il s'y crée une dépression qui tend à aspirer du fluide hydraulique depuis le ballonnet 10 de la manchette 1, par l'intermédiaire du tube 8, du bloqueur 4 (en position communicante) et du tube 7. Le ballonnet 10 n'étant plus sous pression, le conduit urinaire n'est plus comprimé et la miction est rendue possible.

A la fin de l'opération, le retour à l'état de repos se fait comme déjà indiqué plus haut : annulation de la force F, retour de fluide du ballonnet 14 vers l'organe d'actionnement 3, rapprochement des plateaux 12 et 13, diminution du volume de la chambre 17, qui revient en communication avec la chambre 21, et augmentation du volume du ballonnet 10 de la manchette 1, qui comprime le conduit urinaire 26.

Enfin, comme indiqué plus haut, la présence d'un bloqueur 4 permet le maintien du patient dans l'état d'incontinence : après application de la force F, il suffit de mettre le bloqueur 4 en position de circuit coupé pour que tout retour de fluide hydraulique vers le ballonnet 10 soit interdit.

Les matériaux qui constituent le dispositif de commande selon l'invention sont facilement déterminés par l'homme du métier, compte tenu des qualités mécaniques et physiologiques demandées à chaque élément qui le constituent, et compte tenu de leur mode de fabrication, qui est essentiellement le moulage. Ainsi le ballonnet 14 est en un matériau souple et non élastique, par exemple en polyéthylène ; les plateaux 12 et 13 et la tige de butée 25 sont en un matériau rigide, par exemple en polytérephtalate d'éthylène ; le clapet 24 est en un matériau semi-rigide, par exemple, en silicone ; la bague de rappel 18 est en matériau élastique, par exemple en silicone ; le soufflet 16 est une membrane souple et élastique, par exemple en silicone ; le boîtier 19 est en un matériau rigide ou semi-rigide, et le ballon de réserve 20 est en un matériau souple et élastique et, étant soudés l'un à l'autre, ils sont tous deux par exemple en silicone ; les tubes 6 et 7 sont en un matériau souple et non élastique, par exemple en silicone.

Il va de soi que le terme générique "silicone" employé ici ne désigne que la nature chimique d'un matériau, et qu'il s'applique tant à des matériaux rigides qu'à des matériaux souples ou élastiques, parmi lesquels l'homme de l'art saura choisir.

Les dimensions du dispositif de commande sont déterminées en fonction du volume de fluide hydraulique à injecter ou à évacuer de la manchette 1, pour gonfler ou dégonfler le ballonnet 10. Ainsi, par exemple, pour un volume de fluide échangé de l'ordre du millilitre, la longueur hors tout du dispositif de commande peut être de 60 à 80 mm, les autres dimensions étant proportionnelles à celles suggérées par le dessin annexé.

Enfin, le fluide hydraulique, qui est mis en oeuvre dans le dispositif de commande et dans l'ensemble de la prothèse, doit être évidemment un liquide physiologiquement acceptable et iso-osmotique au milieu intérieur, tel que par exemple du sérum physiologique.

## Revendications

1. Dispositif de commande pour un sphincter artificiel d'un conduit naturel, notamment urinaire, ledit sphincter comportant une enveloppe de volume variable, ledit dispositif comprenant une première chambre (14), contenant un fluide incompressible, en communication avec un organe d'actionnement (3) permettant de faire varier le volume de ladite première chambre (14), une deuxième chambre (17), contenant un fluide incompressible, en communication avec ledit sphincter, lesdites première et deuxième chambres étant agencées de sorte qu'une variation de volume de ladite première chambre entraîne une variation de volume, de même sens, de ladite deuxième chambre, ledit dispositif pouvant passer, par actionnement dudit organe (3), d'un premier état, dans lequel les volumes desdites première et deuxième chambres sont minimaux, le sphincter obturant alors ledit conduit naturel, à un second état, dans lequel les volumes desdites première et deuxième chambres sont maximaux, les sphincter ouvrant alors ledit conduit, des moyens élastiques de rappel (18) étant prévus pour ramener spontanément ledit dispositif dudit second état audit premier état,
caractérisé en ce que ladite première chambre est un ballonnet (14), en ce que ladite deuxième chambre (17) est définie par deux plateaux (12,13), reliés par une membrane formant soufflet (16), entre lesquels est logé ledit ballonnet (14), et en ce que lesdits moyens élastiques de rappel comprennent au moins une bague élastique (18) entourant lesdits plateaux (12,13).

2. Dispositif selon la revendication 1,
caractérisé en ce que lesdites première et deuxième chambres sont logées dans un boîtier rigide ou semi-rigide (19) communiquant avec un ballon de réserve (20) par un orifice (23) ménagé dans la paroi du boîtier en définissant ainsi une troisième chambre (21), et en ce qu'il est prévu entre lesdites deuxième et troisième chambres des moyens de liaison fluidique (22,24,25) permettant de mettre en communication lesdites deuxième (17) et troisième (21) chambres lorsque lesdits plateaux (12,13) sont rapprochés l'un de l'autre, et d'isoler lesdites deuxième (17) et troisième (21) chambres l'une de l'autre lorsque lesdits plateaux (12,13) sont éloignés l'un de l'autre.

3. Dispositif selon la revendication 2,
caractérisé en ce que lesdits moyens de liaison fluidique entre lesdites deuxième (17) et troisième (21) chambres comprennent un orifice (22), ménagé dans l'un (12) desdits plateaux, pouvant être ouvert ou fermé grâce à un clapet mobile (24) qui ferme le passage lorsque lesdits plateaux (12,13) sont écartés l'un de l'autre, et qui ouvre le passage, en venant buter contre une tige (25) solidaire de l'autre (13) desdits plateaux, lorsque lesdits plateaux (12,13) se rapprochent l'un de l'autre.

4. Dispositif selon l'une quelconque des revendications 1 à 3,
caractérisé en ce que lesdits plateaux (12,13) présentent, sur leurs faces en regard l'une de l'autre, des reliefs assurant le positionnement du ballonnet (14).

5. Dispositif selon la revendication 4,
caractérisé en ce que, le ballonnet (14) ayant une forme sensiblement cylindrique à l'état gonflé, l'un (13) des plateaux comporte un évidement en forme de gouttière tandis que l'autre (12) desdits plateaux présente un relief de forme convexe conjuguée à celle dudit évidement, pour le positionnement dudit ballonnet (14).

6. Dispositif selon l'une quelconque des revendications 1 à 5,
caractérisé en ce que ledit organe d'actionnement (3) comporte un réservoir souple de fluide incompressible (5).

7. Dispositif selon l'une quelconque des revendications 1 à 6,
caractérisé en ce qu'il comprend un organe de blocage (4) permettant d'interrompre, temporairement, la communication fluidique entre ledit sphincter et ladite deuxième chambre.

8. Dispositif selon la revendication 7,
caractérisé en ce que ledit organe de blocage (4) comprend un corps en matériau élastique contenant deux chambres sensiblement sphériques en communication l'une avec l'autre, et une bille susceptible d'occuper l'une ou l'autre desdits chambres.

9. Dispositif selon la revendication 8,
caractérisé en ce que ledit corps est solidaire dudit organe d'actionnement (3).

10. Prothèse implantable comportant un sphincter artificiel d'un conduit naturel, notamment urinaire, ledit sphincter présentant une enveloppe de volume variable, caractérisée en ce qu'elle comprend un dispositif de commande selon l'une quelconque des revendications 1 à 9, dont la deuxième chambre (17) est reliée, par une liaison fluidique, audit sphincter (1).

## Patentansprüche

1. Steuervorrichtung für einen künstlichen Schließmuskel eines natürlichen Kanals, insbesondere eines Harnleiters, wobei der künstliche Schließmuskel eine Hülle mit variablem Volumen aufweist und wobei die Vorrichtung eine erste, eine inkompressible Flüssigkeit enthaltende Kammer (14), die in Verbindung mit einem Betätigungsorgan (3) steht, das es erlaubt, das Volumen der ersten Kammer (14) zu variieren und eine zweite Kammer (17) umfaßt, die eine inkompressible Flüssigkeit enthält und mit dem künstlichen Schließmuskel in Verbindung steht, wobei die erste und die zweite Kammer derart angeordnet sind, daß eine Volumenveränderung der ersten Kammer eine Volumenveränderung der zweiten Kammer im gleichen Sinn bewirkt, wobei die Vorrichtung mittels der Betätigung des Organs (3) aus einem ersten Zustand, in dem die Volumina der ersten und der zweiten Kammer minimal sind und der künstliche Schließmuskel den natürlichen Kanal verschließt, in einen zweiten Zustand übergehen kann, in dem die Volumina der ersten und zweiten Kammer maximal sind und der künstliche Schließmuskel nun den Kanal öffnet, wobei elastische Rückstellmittel (18) vorgesehen sind, um die Vorrichtung spontan aus dem zweiten Zustand in den ersten Zustand zurückzuführen, dadurch gekennzeichnet, daß die erste Kammer ein Ballon (14) ist, daß die zweite Kammer (17) durch zwei Platten (12, 13) gebildet wird, die miteinander durch eine einen Balg (16) bildende Membran verbunden sind und zwischen denen der Ballon (14) angeordnet ist, und dadurch, daß die elastischen Rückstellmittel wenigstens einen elastischen Ring (18) umfassen, der die Platten (12, 13) umschließt.

2. Vorrichtung nach Anspruch 1, dadurch gekennzeichnet, daß die erste und zweite Kammer in einem starren oder halbstarren Gehäuse (19) untergebracht sind, das mit einem Reserveballon (20) über eine in der Seitenwand des Gehäuses angebrachte Öffnung (23) in Verbindung steht, der somit eine dritte Kammer (21) bildet, und dadurch, daß zwischen der zweiten und der dritten Kammer fluidtechnische Verbindungsmittel (22, 24, 25) vorgesehen sind, die es erlauben, die zweite (17) und dritte (21) Kammer zu verbinden, wenn die Platten (12, 13) einander angenähert sind und die zweite (17) und dritte (21) Kammer voneinander zu trennen, wenn die Platten (12, 13) voneinander entfernt sind.

3. Vorrichtung nach Anspruch 2, dadurch gekennzeichnet, daß die fluidtechnischen Verbindungsmittel zwischen der zweiten (17) und der dritten (21) Kammer eine Öffnung (22) umfassen, die in einer (12) der Platten angebracht ist und die mittels einer beweglichen Klappe (24) geöffnet oder geschlossen werden kann, wobei die bewegliche Klappe (24) den Durchgang schließt, wenn die Platten (12, 13) voneinander entfernt sind, und den Durchgang öffnet, wenn die Platten (12, 13) sich einander annähern, wobei sie an eine feste Stange (25) der anderen (13) der Platten anschlägt.

4. Vorrichtung nach einem der Ansprüche 1 bis 3, dadurch gekennzeichnet, daß die Platten (12, 13) auf ihren einander zugewandten Seiten Erhebungen aufweisen, die die Positionierung des Ballons (14) sicherstellen.

5. Vorrichtung nach Anspruch 4, dadurch gekennzeichnet, daß eine (13) der Platten eine rinnenförmige Aussparung aufweist, während die andere (12) der Platten eine dieser Aussparung entsprechende Erhebung von konvexer Form für die Positionierung des Ballons (14) aufweist, wobei der Ballon (14) eine ungefähr zylindrische Form in aufgeblasenem Zustand aufweist.

6. Vorrichtung nach einem der Ansprüche 1 bis 5, dadurch gekennzeichnet, daß das Betätigungsorgan (3) ein nachgiebiges Reservoir inkompressibler Flüssigkeit (5) aufweist.

7. Vorrichtung nach einem der Ansprüche 1 bis 6, dadurch gekennzeichnet, daß sie ein Verriegelungsorgan (4) umfaßt, das es erlaubt, die fluidtechnische Verbindung zwischen dem künstlichen Schließmuskel und der zweiten Kammer zeitweise zu unterbrechen.

8. Vorrichtung nach Anspruch 7, dadurch gekennzeichnet, daß das Verriegelungsorgan (4) einen Körper aus elastischem Material umfaßt, der zwei im wesentlichen sphärische, miteinander in Verbindung stehende Kammern sowie eine Kugel umfaßt, die dafür geeignet ist, die eine oder die andere Kammer zu besetzen.

9. Vorrichtung nach Anspruch 8, dadurch gekennzeichnet, daß der Körper fest mit dem Betätigungsorgan (3) verbunden ist.

10. Implantierbare Prothese mit einem künstlichen Schließmuskel eines natürlichen Kanals, insbesondere eines Harnleiters, wobei der künstliche Schließmuskel eine hülle mit variablem Volumen aufweist, dadurch gekennzeichnet, daß sie eine Steuervorrichtung nach einem der Ansprüche 1 bis 9 umfaßt, deren zweite Kammer (17) über eine fluidtechnische Verbindung mit dem künstlichen Schließmuskel (1) verbunden ist.

## Claims

1. Control device for an artificial sphincter of a natural, in particular urinary, canal, the said sphincter comprising a variable-volume envelope, the said device comprising a first chamber (14), containing an incompressible fluid, connecting with an actuation member (3) allowing variation of the volume of the said first chamber (14), a second chamber (17), containing an incompressible fluid, connecting with the said sphincter, the said first and second chambers being arranged so that a variation in volume of the said first chamber leads to a variation in volume, of the same sense, of the said second chamber, the said device being able to pass, by actuating the said member (3), from a first state, in which the volumes of the said first and second chambers are minimal, the sphincter then closing the said natural canal, to a second state, in which the volumes of the said first and second chambers are maximal, the sphincter then opening the said canal, elastic return means (18) being provided in order spontaneously to return the said device from the said second state to the said first state, characterised in that the said first chamber is a balloon (14), in that the said second chamber (17) is defined by two plates (12, 13), connected by a membrane forming a bellows (16), between which the said balloon (14) is housed, and in that the said elastic return means comprise at least one elastic ring (18) surrounding the said plates (12, 13).

2. Device according to Claim 1, characterised in that the said first and second chambers are housed in a rigid or semi-rigid case (19) connected with a reserve ball (20) via an orifice (23) made in the wall of the case and thus defining a third chamber (21), and in that there are provided between the said second and third chambers means for fluid linkage (22, 24, 25) allowing the said second (17) and third (21) chambers to be connected when the said plates (12, 13) are brought together, and to isolate the said second (17) and third (21) chambers from each other when the said plates (12, 13) are separated from each other.

3. Device according to Claim 2, characterised in that the said means for fluid linkage between the said second (17) and third (21) chambers comprise an orifice (22) made in one (12) of the said plates, which can be opened or closed using a moving flap (24) which closes the passage when the said plates (12, 13) are separated from each other, and which opens the passage, coming to abut against a rod (25) integral with the other (13) of the said plates, when the said plates (12, 13) are brought together.

4. Device according to any one of Claims 1 to 3, characterised in that the said plates (12, 13) have, on their faces facing each other, reliefs ensuring positioning of the balloon (14).

5. Device according to Claim 4, characterised in that, the balloon (14) having a substantially cylindrical shape in the inflated state, one (13) of the plates comprises a gutter-shaped recess whereas the other (12) of the said plates has a relief of convex shape conjugate with that of the said recess, for positioning the said balloon (14).

6. Device according to any one of Claims 1 to 5, characterised in that the said actuation member (3) comprises a flexible reservoir of incompressible fluid (5).

7. Device according to any one of Claims 1 to 6, characterised in that it comprises a blocking member (4) allowing the fluid connection between the said sphincter and the said second chamber to be temporarily interrupted.

8. Device according to Claim 7, characterised in that the said blocking member (4) comprises a body made of an elastic material containing two substantially spherical chambers connecting with each other, and a small ball capable of occupying one or other of the said chambers.

9. Device according to Claim 8, characterised in that the said body is integral with the said actuation member (3).

10. Implantable prosthesis comprising an artificial sphincter of a natural, in particular urinary, canal, the said sphincter having a variable-volume envelope, characterised in that it comprises a control device according to any one of Claims 1 to 9, whose second chamber (17) is connected, by a fluid linkage, to the said sphincter (1).
